Europäisches Patentamt

European Patent Office

Office européen des brevets

(11) Veröffentlichungsnummer: **0 316 761**
A2

## EUROPÄISCHE PATENTANMELDUNG

(21) Anmeldenummer: 88118720.7

(22) Anmeldetag: 10.11.88

(51) Int. Cl.⁴: **C07C 85/12** , **C07C 87/18**

(30) Priorität: 18.11.87 DE 3739260

(43) Veröffentlichungstag der Anmeldung:
24.05.89 Patentblatt 89/21

(84) Benannte Vertragsstaaten:
CH DE FR GB LI

(71) Anmelder: **BAYER AG**
**Konzernverwaltung RP Patentabteilung**
**D-5090 Leverkusen 1 Bayerwerk(DE)**

(72) Erfinder: **Kiel, Wolfgang, Dr.**
**Carl-Leverkus-Strasse 37**
**D-5068 Odenthal(DE)**
Erfinder: **Bauer, Wolfgang, Dr.**
**Karl-Krekeler-Strasse 17**
**D-5090 Leverkusen 1(DE)**

(54) Verfahren zur Herstellung von N,N-Dimethyldiaminopropan.

(57) N,N-Dimethyldiaminopropan mit besonders geringem Gehalt an 1,3-Diaminopropan wird durch katalytische Hydrierung von N,N-Dimethylaminopropionitril, gegebenenfalls in Gegenwart von Ammoniak erhalten, wenn man in Gegenwart eines oder mehrer Erdalkalioxide arbeitet.

EP 0 316 761 A2

## Verfahren zur Herstellung von N,N-Dimethyldiaminopropan

Die vorliegende Erfindung betrifft ein Verfahren zur Herstellung von N,N-Dimethyldiaminopropan-(1,3) durch Hydrierung von N,N-Dimethylaminopropionitril, wobei ein Produkt mit besonders niedrigem Gehalt an 1,3-Diaminopropan erhalten wird.

N,N-Dimethyldiaminopropan ist ein wichtiges Zwischenprodukt zur Herstellung von Arzneimitteln, von oberflächenaktiven Substanzen, von Härtern für Epoxyharze, von Ionenaustauschern und von Polyurethanen.

Es ist bekannt, daß man N,N-Dimethyldiaminopropan herstellen kann, indem man N,N-Dimethylaminopropionitril katalytisch in Gegenwart von Ammoniak hydriert und das Hydriergemisch anschließend destilliert. N,N-Dimethylaminopropionitril ist seinerseits gut zugänglich durch Addition von Dimethylamin an Acrylnitril.

Das auf diese Weise hergestellte N,N-Dimethyldiaminopropan enthält im allgemeinen einige Verunreinigungen, wobei 1,3-Diaminopropan besonders unerwünscht ist, weil es einerseits durch seine Bifunktionalität stört und andererseits mit einem Siedepunkt von 136° C (bei Normaldruck) destillativ praktisch nicht vom N,N-Dimethyldiaminopropan (Siedepunkt 134 bis 135° C bei Normaldruck) abgetrennt werden kann. Die Bifunktionalität des 1,3-Diaminopropans macht sich insbesondere dann nachteilig bemerkbar, wenn es in Konzentrationen von über 100 ppm in N,N-Dimethyldiaminopropan vorhanden ist, weil dann bei Folgeumsetzungen mit solchem N,N-Dimethyldiaminopropan Vernetzungen und/oder Gelbildungen auftreten.

Bisher ist lediglich bekanntgeworden, daß man 1,3-Diaminopropan aus N,N-Dimethyldiaminopropan entfernen kann, indem man die nach der Hydrierung durchzuführende Destillation in Gegenwart eines Übergangsmetallsalzes durchführt, das mit dem 1,3-Diaminopropan einen stabilen Komplex ergibt (siehe EP-OS 0 065 709). Ein geeignetes Übergangsmetallsalz für diesen Zweck ist beispielsweise Nickelsulfat. Nachteilig hierbei ist, daß die Bildung von 1,3-Diaminopropan nicht vermieden wird und Probleme bei der Abtrennung und Entsorgung der gebildeten 1,3-Diaminopropan-Übergangsmetallsalz-Komplexe auftreten. Es besteht deshalb nach wie vor das Bedürfnis nach einem Herstellungsverfahren für N,N-Dimethyldiaminopropan, bei dem von vornherein nur sehr wenig 1,3-Diaminopropan gebildet wird.

Es wurde nun ein Verfahren zur Herstellung von N,N-Dimethyldiaminopropan-1,3 durch katalytische Hydrierung von N,N-Dimethylaminopropionitril, gegebenenfalls in Gegenwart von Ammoniak gefunden, das dadurch gekennzeichnet ist, daß man die Hydrierung in Gegenwart eines oder mehrerer Erdalkalioxide vornimmt.

Als Katalysatoren für die katalytische Hydrierung kommen übliche Hydrierkatalysatoren in Frage, beispielsweise solche auf der Basis von Nickel oder Kobalt. Diese Metalle können als solche vorliegen, beispielsweise in Form von Raney-Metallen, sie können aber auch auf Trägern aufgebracht sein. Bevorzugt gelangt Raney-Kobalt zum Einsatz.

Geeignete Reaktionsbedingungen für die katalytische Hydrierung sind beispielsweise Temperaturen im Bereich von 100 bis 250° C und Drucke im Bereich von 10 bis 200 bar. Die Hydrierung kann beispielsweise in Gegenwart von 0 bis 20 Gew.-% Ammoniak (bezogen auf den gesamten Ansatz) durchgeführt werden, wobei man vorzugsweise in Gegenwart von Ammoniak arbeitet und diesen im allgemeinen in reiner Form, gasförmig oder flüssig zuführt.

Erfindungsgemäß führt man die Hydrierung in Gegenwart eines oder mehrerer Erdalkalioxide durch. Hierfür kommen beispielsweise die Oxide des Magnesiums, Calciums, Strontiums und/oder Bariums in Frage. Vorzugsweise wird Magnesiumoxid und/oder Calciumoxid eingesetzt.

Die Erdalkalioxide können beispielsweise in Mengen von 0,001 bis 10 Gew.-%, bezogen auf das gesamte Reaktionsgemisch, verwendet werden. Vorzugsweise beträgt diese Menge 0,005 bis 5 Gew.-%, besonders bevorzugt 0,001 bis 3 Gew.-%.

Man kann das erfindungsgemäße Verfahren sowohl kontinuierlich als auch diskontinuierlich durchführen. Bei einer diskontinuierlichen Durchführung kann man z.B. so verfahren, daß man in einem Autoklaven N,N-Dimethylaminopropionitril, Katalysator, Ammoniak und Erdalkalimetalloxid vorlegt, dann unter Rühren z.B. 25 bis 80 bar Wasserstoff aufdrückt, das Gemisch bis zur Anspringtemperatur des Katalysators aufheizt, dann den Wasserstoffdruck gegebenenfalls erhöht (z.B. auf 100 bis 200 bar) und eine Reaktionstemperatur beispielsweise im Bereich von 120 bis 250° C einstellt. Nach Beendigung der Wasserstoffaufnahme kann man noch eine gewisse Zeit auf Temperaturen im Bereich 120 bis 250° C halten, beispielsweise für 5 bis 60 Minuten. Danach kann man abkühlen, entspannen, feste Bestandteile des Reaktionsgemisches (z.B den Katalysator) abfiltrieren und aus dem Filtrat durch Destillation das gebildete N,N-Dimethyldiaminopropan-1,3 gewinnen.

2

Das erfindungsgemäße Verfahren liefert ein N,N-Dimethyldi-aminopropan-1,3, das in der Regel weniger als 100 ppm, häufig weniger als 50 ppm 1,3-Diaminopropan enthält. Wesentlich ist, daß nur derartig geringe Mengen 1,3-Diaminopropan während der Hydrierung gebildet werden und deshalb ein besonderer Aufwand für die Entfernung von einmal gebildetem 1,3-Diaminopropan entfällt. Diese Ergebnisse sind ausgesprochen überraschend, da die Hydrierung ohne Erdalkalioxid-Zusätze Produkte mit Gehalten von beispielsweise bis zu 430 ppm 1,3-Diaminopropan und die Hydrierung mit Zusätzen von anderen Metalloxiden oder mit Zusätzen von anderen Erdalkaliverbindungen Produkte mit Gehalten von beispielsweise bis zu 2500 ppm 1,3-Diaminopropan liefert (siehe die vorliegenden Vergleichsbeispiele).

Beispiele

Alle Beispiele wurden wie folgt durchgeführt: ·

In einem 0,7 1-Stahlautoklaven wurden 200 g N,N-Dimethylaminopropionitril, 6 g Raney-Kobalt, die jeweils angegebene Menge Zusatzstoff und 20 g NH₃ (flüssig) vorgelegt. Dann wurde 50 bar Wasserstoff aufgedrückt und erhitzt, bis die Hydrierung begann. Dann wurde ein Wasserstoffdruck von 150 bar und die jeweils angegebene Reaktionstemperatur eingestellt. Nach Beendigung der Wasserstoffaufnahme wurden noch 20 Minuten lang Druck und Temperatur gehalten. Danach wurde abgekühlt, entspannt, der Katalysator abfiltriert und im verbleibenden Gemisch der Gehalt an 1,3-Diaminopropan gaschromatographisch bestimmt. Die Einzelergebnisse sind aus der folgenden Tabelle 1 ersichtlich.

Tabelle 1

| Beispiel Nr. | Zusatzstoff | Reaktionstemperatur | Gehalt an 1,3-Diaminopropan |
|---|---|---|---|
| 1 | 6 g CaO | 160° C | <50 ppm |
| 2 | 1 g CaO | 180° C | 50 ppm |
| 3 | 0,1 g CaO | 160° C | <50 ppm |
| 4 | 0,1 g MgO | 160° C | 70 ppm |
| 5 | - | 160° C | 220 ppm |
| 6 | - | 180° C | 430 ppm |
| 7 | 1 g Al₂O₃ | 160° C | 180 ppm |
| 8 | 1 g CaCl₂ | 160° C | 2500 ppm |
| 9 | 1 g MgSO₄ | 160° C | 110 ppm |
| 10 | 1 g Na₂SO₄ | 160° C | 120 ppm |
| 11 | 0,3 g NiSO₄ | 140° C | 130 ppm |
| 12 | - | 130° C | 200 ppm |

Die Beispiele 5 bis 12 dienen zum Vergleich.

**Ansprüche**

1. Verfahren zur Herstellung von N,N-Dimethyldiaminopropan-1,3 durch katalytische Hydrierung von N,N-Dimethylaminopropionitril, gegebenenfalls in Gegenwart von Ammoniak, dadurch gekennzeichnet, daß man die Hydrierung in Gegenwart eines oder mehrerer Erdalkalioxide vornimmt.

2. Verfahren nach Anspruch 1, dadurch gekennzeichnet, daß man die Hydrierung bei Temperaturen im Bereich von 100 bis 250° C und Drucken im Bereich von 10 bis 200 bar durchführt, sowie in Gegenwart von 0 bis 20 Gew.-% Ammoniak (bezogen auf den gesamten Ansatz).

3. Verfahren nach Ansprüchen 1 und 2, dadurch gekennzeichnet, daß man in Gegenwart von Magnesiumoxid, Calciumoxid, Strontiumoxid und/oder Bariumoxid arbeitet.

4. Verfahren nach Ansprüchen 1 bis 3, dadurch gekennzeichnet, daß man in Gegenwart von Magnesiumoxid und/oder Calciumoxid arbeitet.

5. Verfahren nach Ansprüchen 1 bis 4, dadurch gekennzeichnet, daß 0,001 bis 10 Gew.-% Erdalkalimetalloxide vorliegen, bezogen auf das gesamte Reaktionsgemisch.